# EUROPEAN PATENT APPLICATION

(11) **EP 1 072 558 A1**
(43) Date of publication of application: **31.01.2001**
(21) Application number: 00115755.1
(22) Date of filing: 21.07.2000
(51) Int. Cl.: C02F 1/32

(54) **Ultraviolet liquid treatment apparatus**

(30) Priority: 26.07.1999 JP 21127199
(71) Applicant: Photoscience Japan Corporation, Hachioji-shi, Tokyo (JP)
(72) Inventor: Nakano, Koji, c/o Photoscience Japan Corporation, Hachioji-shi, Tokyo (JP); Yamakoshi, Yuji, c/o Photoscience Japan Corp., Hachioji-shi, Tokyo (JP)
(74) Representative: Kehl, Günther, Dipl.-Phys.

(57) **Abstract**

In an ultraviolet liquid treatment apparatus, a lamp protecting pipe (4) with an ultraviolet lamp (5) accommodated therein is brought into contact with a liquid to be treated, so as to treat the liquid with ultraviolet rays irradiated by the ultraviolet lamp. Ballast (8) for operating the ultraviolet lamp is provided within the lamp protecting pipe (4) or outside the lamp protecting pipe in proximate relation thereto. With such arrangements, the ballast can be effectively cooled by the liquid to be treated and also can be reduced in size.

## Description

The present invention relates generally to ultraviolet liquid treatment apparatus which use ultraviolet irradiation to perform liquid treatment, such as sterilization or disinfection of liquid, killing of bacteria in the liquid, oxidation-decomposition of a slight amount of organic substances in the liquid or decomposition of decomposition-retardant (hard-to-decompose) organic substances in the liquid. More particularly, the present invention relates to an improved arrangement of a ballast for operating an ultraviolet lamp in ultraviolet liquid treatment apparatus.

Ultraviolet liquid treatment apparatus or systems for performing liquid treatment, such as sterilization or disinfection of liquid and killing of bacteria in the liquid by ultraviolet irradiation, have been used in a variety of fields including pure water production lines in the semiconductor industry, sterilized water production lines in the pharmaceutical industry, water sterilization lines in the food industry, water sterilization lines in cultivation of fishes, shellfishes etc., and waste water/sewage treatment lines. Generally, each ultraviolet lamp used in such liquid treatment is accommodated in a hard, transparent lamp protecting pipe, made of quartz glass, in a liquid-tight manner, and the lamp protecting pipe with the ultraviolet lamp accommodated therein is soaked in a liquid to be treated. Such ultraviolet liquid treatment apparatus or systems are classified into two major types: an open-water-path type where each ultraviolet lamp is placed in an open water path through which the liquid to be treated is caused to flow; and a closed-water-path (sealed tube) type where each ultraviolet lamp is placed in a closed water path (sealed tube) through which the liquid to be treated is caused to flow. The ultraviolet lamp is lit by being supplied with predetermined high-frequency signals that are generated from a commercial A.C. power supply via a lamp illuminating ballast.

In the conventionally-known ultraviolet liquid treatment apparatus, a plurality of the lamp illuminating ballasts are provided within a power supply box in spaced-apart relation to the corresponding ultraviolet lamps. The high-frequency signals output via the individual illuminating ballasts, provided in corresponding relation to the ultraviolet lamps, are fed via respective electric wires or lines to the corresponding ultraviolet lamps. In those apparatus of the above-mentioned sealed tube type, maintenance covers are provided at opposite ends of the sealed tube through which the liquid to be treated is caused to flow, and each of these maintenance covers defines an interior space that is shut off from the interior of the sealed tube in a liquid-tight manner but kept in communication with an interior space of each of the lamp protecting pipes. Within each of the maintenance covers, the electric lines extending from each of the illuminating ballasts are electrically connected via sockets to the corresponding ultraviolet lamp accommodated in the protecting pipe. Further, each of the ultraviolet lamps can be replaced with another one by detaching/attaching the sockets at the positions of the maintenance covers.

Because the lamp illuminating ballasts produce relatively high heat during illumination of the ultraviolet lamps, reliable measures must be taken for heat radiation. For this purpose, it has been conventional to provide heat radiating fins or plates for each of the illuminating ballasts and also provide a cooling fan in the power supply box accommodating these illuminating ballasts. The provision of the radiating fins or plates and cooling fan would unavoidably increase the overall size of the power supply box to a considerable degree. Further, the cooling fan, blowing air within the power supply box, tends to produce dust dirt, which would thus require periodic maintenance. Such periodic maintenance would also be required for a longer life of the cooling fan. Furthermore, because the lines extending from the power supply box to the individual ultraviolet lamps are externally-exposed high-frequency output lines from the illuminating ballasts, there would also produce undesired high-frequency noise. Besides, In the liquid treatment apparatus of the sealed tube type, where the ultraviolet lamp replacement is effected by temporarily detaching the sockets from the lamp, the sockets must be detached/attached from/to the opposite ends of the lamp while ascertaining that the sockets are of a same socket number. Thus, in the case where the apparatus is equipped with a great number of the ultraviolet lamps and when many of the ultraviolet lamps are to be replaced at the same time, cumbersome and time-consuming work would be required for matching the socket numbers.

It is therefore an object of the present invention to provide an ultraviolet liquid treatment apparatus which can appropriately overcome at least one of the above-discussed problems. Namely, the present invention seeks to provide an ultraviolet liquid treatment apparatus which can be reduced in its overall size by improved heat radiation from an illuminating ballast.

In order to accomplish the above-mentioned object, the present invention provides an ultraviolet liquid treatment apparatus which comprises: an ultraviolet lamp accommodated in a light-transmissive lamp protecting pipe, the lamp protecting pipe with the ultraviolet lamp accommodated therein being brought into contact with a liquid to be treated, so as to treat the liquid with ultraviolet rays irradiated by the ultraviolet lamp; and a ballast for operating the ultraviolet lamp, characterized in that said ballast is provided within the lamp protecting pipe or outside the lamp protecting pipe in proximate relation thereto.

Because the ballast for operating or illuminating the ultraviolet lamp accommodated in the protecting pipe is provided within the lamp protecting pipe or outside the lamp protecting pipe in proximate relation thereto, the ballast can be cooled by being located close to a liquid-contacting region of the lamp protecting pipe, and thus heat radiation from the ballast can be promoted. Accordingly, separate heat radiation means for the ballast can be dispensed with or reduced in size, so that the ballast can be reduced in size. Further, the omission or downsizing of the heat radiation means can effectively avoid the necessity for the ballast to be provided within a power supply box, for the purpose of heat radiation, as has been the case with the conventionally-known ultraviolet liquid treatment apparatus. That is, the present invention allows the ballast to be provided in or close to the lamp protecting pipe, in spaced apart relation to the power supply box, rather than in the power supply box, thereby considerably downsizing the power supply box as compared to the conventional counterparts. As a result, the ultraviolet liquid treatment apparatus of the present invention, which does not require a large installation space for the power supply box, can be appropriately installed even in a small space and provides improved convenience of use.

According to one embodiment of the present invention, the lamp protecting pipe with the ultraviolet lamp accommodated therein in a liquid-tight manner is accommodated in a sealed tube through which the liquid to be treated is caused to flow, and an end cover is provided on at least one end of the sealed tube and defines an interior space that is shut off from the interior of the sealed tube in a liquid-tight manner but communicates with an interior space of the lamp protecting pipe. Here, the ballast is provided in the interior space of the end cover. In this case, the ballast does not directly contact the liquid to be treated in the sealed tube, but contacts the liquid indirectly via the one end of the sealed tube, so that the ballast can be cooled appropriately by the liquid without a need for heat radiating fins or plates. To supplement the cooling effect, there may be provided a cooling fan at a suitable location of the end cover (maintenance cover), in which case a small-size cooling fan will suffice.

According to another embodiment of the present invention, the lamp protecting pipe with the ultraviolet lamp accommodated therein in a liquid-tight manner is accommodated in the sealed tube, and the ballast is provided within the sealed tube. In this case, the ballast directly contacts the liquid to be treated in the sealed tube and thus can be cooled sufficiently without a need for heat radiating fins or plates or cooling fan.

According to still another embodiment of the present invention, the lamp protecting pipe with the ultraviolet lamp accommodated therein in a liquid-tight manner is accommodated in the sealed tube, and the ballast is provided within the lamp protecting pipe. Because the lamp protecting pipe directly contacts the liquid to be treated in the sealed tube, the ballast provided in the protecting pipe can be cooled as efficiently as in the case where the ballast directly contacts the liquid, without a need for heat radiating fins or plates or cooling fan.

According to yet another embodiment of the present invention, the ballast is provided within the lamp protecting pipe and has attached thereto a socket for connection with the ultraviolet lamp. In this case too, because the lamp protecting pipe directly contacts the liquid to be treated in the sealed tube, the ballast provided in the protecting pipe can be cooled as efficiently as in the case where the ballast directly contacts the liquid, without a need for heat radiating fins or plates or cooling fan. Further, because the ballast is connected to the ultraviolet lamp via the only one socket, there is no need to match numbers of sockets at the opposite ends of the lamp when the lamp is to be replaced with another one, which thereby greatly facilitates the lamp replacement operations.

According to yet another embodiment of the present invention, the ballast is provided at and projects from one end of the lamp protecting pipe to contact the liquid to be treated, and the one end of the lamp protecting pipe is fixed in place via the ballast. In this case too, the ballast directly contacts the liquid to be treated in the sealed tube, so that it can be cooled sufficiently without a need for heat radiating fins or plates or cooling fan.

For better understanding of the object and other features of the present invention, its preferred embodiments will be described in greater detail hereinbelow with reference to the accompanying drawings, in which:
Fig. 1 is a partly-sectional schematic view of an ultraviolet liquid treatment apparatus of the sealed tube type in accordance with a preferred embodiment of the present invention;
Fig. 2 is a partly-sectional schematic view of another embodiment of the present invention;
Figs. 3A and 3B are partly-sectional schematic views of still other embodiments of the present invention;
Figs. 4A - 4C are partly-sectional schematic views of still other embodiments of the present invention; and
Figs. 5A and 5B are partly-sectional schematic views of yet other embodiments of the present invention.

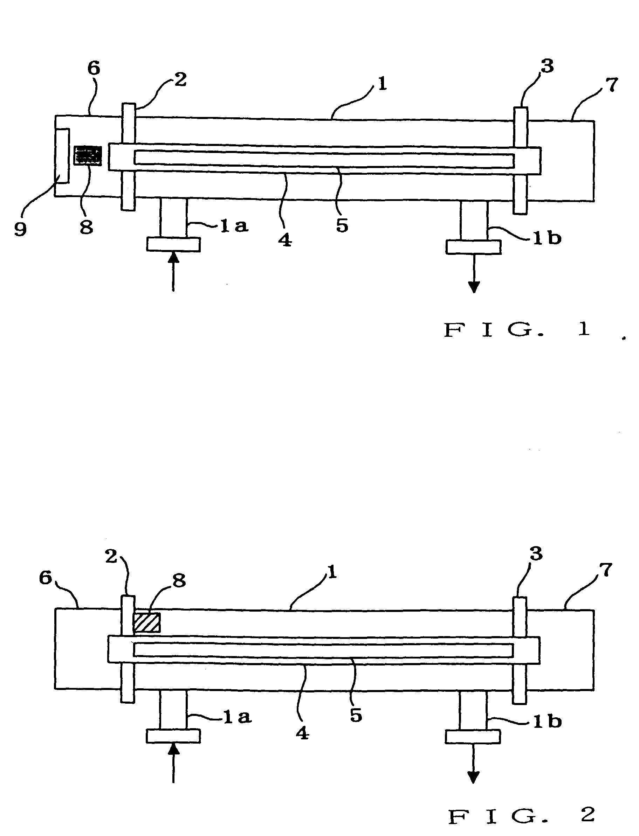

Fig. 1 is a partly-sectional schematic view of an ultraviolet liquid treatment apparatus of the sealed tube type in accordance with a preferred embodiment of the present invention. Sealed tube 1 are sealed, in a liquid-tight manner, at its opposite ends by sealing members 2 and 3 and has a liquid inlet 1a and a liquid outlet 1b. Liquid to be treated is introduced via the inlet 1a, flows longitudinally through the interior of the tube 1, and is discharged via the outlet 1b. Ultraviolet lamp 5 is accommodated in a hard, light-transmissive lamp protecting pipe 4, made of quartz glass, in a liquid-tight manner, and the protecting pipe 4 with the ultraviolet lamp 5 accommodated therein is placed in the sealed tube 1 and soaked in a liquid to be treated so that the ultraviolet lamp 5 acts on the liquid. End covers 6 and 7 mounted on opposite ends of the sealed tube 1 define interior spaces that are shut off, by the respective sealing members 2 and 3, from the interior of the sealed tube 1 in a liquid-tight manner but kept in communication with an interior space of a lamp protecting pipe 4. Namely, opposite ends of the lamp protecting pipe 4 extend through the sealing members 2 and 3 to face the interior spaces of the end covers 6 and 7, respectively. Thus, when the ultraviolet lamp 5 is to be replaced with another one, the ultraviolet lamp 5 can be removed from the lamp protecting pipe 4 through either one of the ends of the protecting pipe 4, and the other or new ultraviolet lamp can be inserted into the protecting pipe 4 through either one of the ends. The end covers 6 and 7 can be opened and closed as desired by means of a known mechanism (not shown); of course, these end covers 6 and 7 are opened when the ultraviolet lamp 5 is to be removed and the new one is to be inserted. Ballast 8 for operating or illuminating the ultraviolet lamp 5 is provided at an appropriate place in the interior space of one of the end covers (end cover 7 in the illustrated example).

The illuminating ballast 8 itself functions in a conventionally-known manner; that is, the illuminating ballast 8 converts a commercial A.C. signal into a D.C. signal and then converts the D.C. signal into a predetermined high-frequency signal for illumination of the ultraviolet lamp 5. Although it has heretofore been necessary to provide heat radiating fins or plates for the conventionally-known illuminating ballast, the instant embodiment can eliminate the need for such heat radiating fins or plates, and accordingly can reduce the size of the illuminating ballast 8, thereby facilitating placement of the illuminating ballast 8 within the cover 6. Specifically, the illuminating ballast 8 in the example of Fig. 1 does not directly contact the liquid contained in the sealed tube 1, but indirectly contact the liquid in the sealed tube 1 by way of the end of the tube 1. More specifically, because air within the cover 6 is cooled by the liquid, there is achieved a considerable cooling effect without heat radiating fins or plates. To increase the cooling effect, it is more preferable to provide a cooling fan 9 at an appropriate place of the end cover 6, in which case the cooling fan 9 may be of a small size as long as it can provide a supplementary cooling effect.

The illuminating ballast 8 has electric wires or lines connected thereto to introduce commercial A.C. signals from the outside. Because the lines are used for introduction of the commercial A.C. signals, there will occur no inconveniences such as high-frequency noise. Lamp-illuminating high-frequency signal generated via the ballast 8 is fed, via electric lines, socket connector and the like, to the ultraviolet lamp 5 accommodated in the lamp protecting pipe 4. In this case, because the ballast 8 is located close to the ultraviolet lamp 5 and positioned within the end cover 6, the undesired high-frequency noise can be minimized. Whereas only one ultraviolet lamp 5 is shown in Fig. 1 for convenience of illustration, a plurality of such ultraviolet lamps 5 accommodated in respective lamp protecting pipes 4 are, in effect, placed in the sealed tube 1; in such a case, a plurality of the ballasts 8 are provided in corresponding relation to the ultraviolet lamps 5. Similarly, in other embodiments to be described later in relation to Figs. 2 - 5, two or more, rather than one, ultraviolet lamps 5 may be provided, and two or more ballasts 8 may be provided in corresponding relation to the ultraviolet lamps 5.

Fig. 2 shows another embodiment of the present invention, where the ballast 8 is provided within the sealed tube 1 for direct contact with the liquid contained in the tube 1. In this case, the electric lines connected to the ballast 8 within the sealed tube 1 extends through the sealing member 2 into the interior space of the end cover 6 and are connected, via a socket connector and the like, to the ultraviolet lamp 5 accommodated in the lamp protecting pipe 4 as well as to an external commercial A.C. power supply. Because, in this case, the ballast 8 directly contacts the liquid to be treated in the sealed tube 1, it can be sufficiently cooled without particular heat radiating fins or plates or cooling fan. Because the ballast 8 itself contacts the liquid, the ballast 8 must of course be enclosed in a package in a liquid-tight manner.

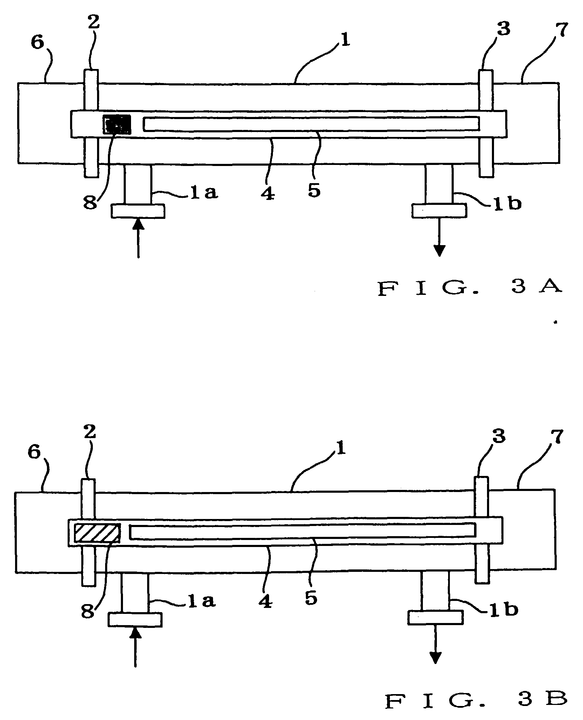

Fig. 3A shows still another embodiment of the present invention, where the ballast 8 is provided within the lamp protecting pipe 4 and extends along a region of the protecting pipe 4 which directly contacts the liquid in the sealed tube 1. In this case, the electric lines connected to the ballast 8 need not necessarily be enclosed in a liquid-tight manner. Namely, the electric lines extending from the external commercial A.C. power supply are introduced into the lamp protecting pipe 4 via one of the pipe's ends and connected to the ballast 8, and the lamp-illuminating high-frequency signal output from the ballast 8 is fed, via electric lines, socket connector and the like, to the ultraviolet lamp 5 accommodated in the lamp protecting pipe 4. Because, in this case, the lamp protecting pipe 4 directly contacts the liquid to be treated in the sealed tube 1, the ballast 8 placed in the protecting pipe 4 can be cooled as efficiently as in the case where the ballast 8 directly contacts the liquid. Thus, there can be provided a sufficient cooling effect without particular heat radiating fins or plates or cooling fan.

The embodiment of Fig. 3A achieves a reliable cooling effect if the ballast 8 is provided within the lamp protecting pipe 4 in such a manner that the entire length of the ballast 8 lies along the liquid-covered region in the sealed tube 1. In a modification, however, the ballast 8 may be provided within the lamp protecting pipe 4 in such a manner that only part of its length extends along the liquid-covered region in the sealed tube 1 and the remaining length of the ballast 8 extends along an end portion of the protecting pipe 4 located in the interior space of the cover 6. In such a case, an enhanced heat radiating efficiency will be achieved if part of the ballast 8, producing higher heat than the remaining part, is positioned to extend along the liquid-covered region in the sealed tube 1. In case the cooling effect is insufficient, a cooling fan 9 may be provided on the cover 6 in the manner as shown in Fig. 1.

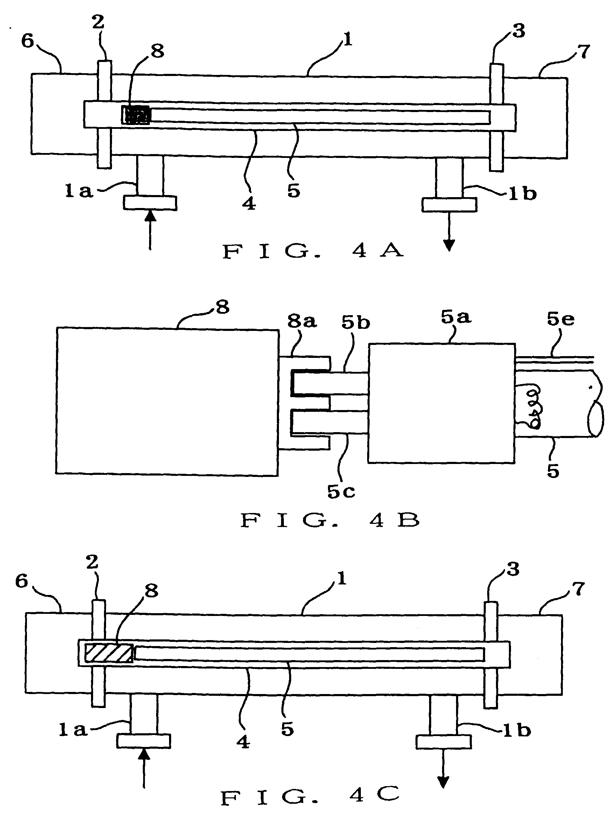

Fig. 4A shows still another embodiment of the present invention, which is similar to the embodiment of Fig. 3A in that the ballast 8 is disposed within the lamp protecting pipe 4 and extends along a region of the protecting pipe 4 which directly contacts the liquid in the sealed tube 1. However, the embodiment of Fig. 4A differs from the embodiment of Fig. 3A in the mechanism for feeding the lamp-illuminating high-frequency signal from the ballast 8 to the ultraviolet lamp 5. Namely, as shown In Fig. 4B on an enlarged scale, the ballast 8 is provided with a socket 8a for connection with the ultraviolet lamp 5, and terminals 5b and 5c mounted on an end cap 5a of the ultraviolet lamp 5 are directly fitted in the socket 8a on the ballast 8. Assuming that the terminal 5b corresponds to an electrode at one end of the ultraviolet lamp 5, the terminal 5c corresponds to an electrode at the other end of the ultraviolet lamp 5, in which case an electric line 5e is connected to the electrode at the other end of the ultraviolet lamp 5 and also connected to the terminal 5c within the cap 5a. Because the ballast 8 is connected to the ultraviolet lamp 5 via the only one socket 8a on the ballast 8, there is no need to match numbers of sockets at the opposite ends of the lamp 5 when the lamp 5 is to be replaced with another one, which thereby greatly facilitates the lamp replacement operations. In this case too, the ballast 8 placed in the protecting pipe 4 can be cooled as efficiently as in the case where the ballast 8 directly contacts the liquid in the sealed tube 1. Thus, there can be provided a sufficient cooling effect without particular heat radiating fins or plates or cooling fan.

The embodiment of Fig. 4A also achieves a reliable cooling effect if the ballast 8 is provided within the lamp protecting pipe 4 in such a manner that the entire length of the ballast 8 lies along the liquid-covered region in the sealed tube 1. In a modification, however, the ballast 8 may be provided within the lamp protecting pipe 4 in such a manner that only part of its length extends along the liquid-covered region in the sealed tube 1 and the remaining length of the ballast 8 extends along an end portion of the protecting pipe 4 located in the interior space of the cover 6. In such a case, an enhanced heat radiating efficiency will be achieved if part of the ballast 8, producing higher heat than the remaining part, is positioned to extend along the liquid-covered region in the sealed tube 1. In case the cooling effect is insufficient, a cooling fan 9 may be provided on the cover 6 in the manner as shown in Fig. 1.

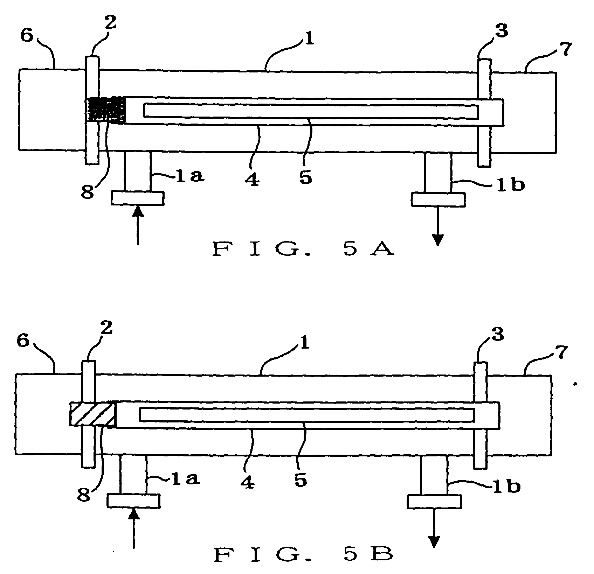

Fig. 5A shows still another embodiment of the present invention, where the ballast 8 is provided at one end of the lamp protecting pipe 4. Specifically, the ballast 8 projects from the one end of the lamp protecting pipe 4 into the sealed tube 1 in an uncovered state and is secured at its distal end to the sealing member 2, so that part of the ballast 8 directly contacts the liquid to be treated. The lamp protecting pipe 4 is secured to the sealing member 2 via the ballast 8. More specifically, the ballast 8 is attached at its one end to one end of the lamp protecting pipe 4 in a liquid-tight manner and detachably for replacement of the ultraviolet lamp 5, and the other end of the ballast 8 extends through the sealing member 2 in a liquid-tight manner to face the interior space of the cover 6; the other end of the ballast 8 is fixed to the sealing member 2 in a liquid-tight manner and detachably for replacement of the ultraviolet lamp 5. Electric lines from the external commercial A.C. power supply extend through the interior space of the cover 6 and sealing member 2 and are coupled to the ballast 8. Electric lines for feeding the lamp-illuminating high-frequency signal from the ballast 8 are connected to the ultraviolet lamp 5 within the lamp protecting pipe 4. Because the ballast 8 directly contacts the liquid to be treated in the sealed tube 1, this embodiment can afford a sufficient cooling effect without particular heat radiating fins or plates or cooling fan. Because the ballast 8 directly contacts the liquid, the ballast 8 must of course be enclosed in a package in a liquid-tight manner.

The embodiment of Fig. 5A also achieves a reliable cooling effect if the ballast 8 is provided in such a manner that the substantially entire length of the ballast 8 lies along the liquid-covered region in the sealed tube 1. In a modification, however, the ballast 8 may be provided in such a manner that only part of its length extends along the liquid-covered region in the sealed tube 1 and the remaining length of the ballast 8 extends into the interior space of the cover 6. In such a case, an enhanced heat radiating efficiency will be achieved if part of the ballast 8, producing higher heat than the remaining part, is positioned to extend along the liquid-contacting region in the sealed tube 1. In case the cooling effect is insufficient, a cooling fan 9 may be provided on the cover 6 in the manner as shown in Fig. 1.

Whereas the preceding paragraphs have described the ultraviolet liquid treatment apparatus of the sealed tube type In relation to several preferred embodiments, it should be appreciated that the basic principles of the present Invention is also applicable to the ultraviolet liquid treatment apparatus of the open-water-path type.

In summary, the present invention is characterized in that the ballast for operating the ultraviolet lamp accommodated in the protecting pipe is provided within the lamp protecting pipe or outside the lamp protecting pipe in proximate relation thereto. Thus, the ballast can be cooled by being located close to the liquid-contacting region of the lamp protecting pipe, so that heat radiation from the ballast can be promoted. Accordingly, separate heat radiation means for the ballast can be dispensed with or reduced in size, so that the ballast can be reduced in size or downsized. Further, the omission or downsizing of the heat radiation means can effectively avoid the necessity for the ballast to be provided within the power supply box, for the purpose of heat radiation, as has been the case with the conventionally-known techniques. That is, the present invention allows the ballast to be provided in or close to the lamp protecting pipe rather than in the power supply box, thereby considerably downsizing the power supply box as compared to the conventional counterparts. As a result, the ultraviolet liquid treatment apparatus of the present invention, which does not require a large installation space for the power supply box, can be appropriately installed even in a small space and provides improved convenience of use.

## Claims

1. An ultraviolet liquid treatment apparatus comprising:
an ultraviolet lamp (5) accommodated in a light-transmissive lamp protecting pipe (4), said lamp protecting pipe with said ultraviolet lamp accommodated therein being brought into contact with a liquid to be treated, so as to treat the liquid with ultraviolet rays irradiated by said ultraviolet lamp (5); and
a ballast (8) for operating said ultraviolet lamp (8),
characterized in that said ballast (8) is provided within said lamp protecting pipe (4) or outside said lamp protecting pipe (4) in proximate relation thereto.

2. An ultraviolet liquid treatment apparatus as claimed in claim 1 wherein said lamp protecting pipe (4) with said ultraviolet lamp (5) accommodated therein in a liquid-tight manner is accommodated in a sealed tube (1) through which the liquid to be treated is caused to flow and an end cover (6) is provided on at least one end of said sealed tube and defines an interior space shut off from an interior of said sealed tube in a liquid-tight manner but communicating with an interior space of said lamp protecting pipe (4), and wherein said ballast (8) is provided in the interior space of said end cover (6).

3. An ultraviolet liquid treatment apparatus as claimed in claim 1 wherein said lamp protecting pipe (4) with said ultraviolet lamp (5) accommodated therein in a liquid-tight manner is accommodated in a sealed tube (1) through which the liquid to be treated is caused to flow, and wherein said ballast (8) is provided within said sealed tube (1).

4. An ultraviolet liquid treatment apparatus as claimed in claim 1 wherein said lamp protecting pipe (4) with said ultraviolet lamp (5) accommodated therein in a liquid-tight manner is accommodated in a sealed tube (1) through which the liquid to be treated is caused to flow, and wherein said ballast (8) is provided within said lamp protecting pipe (4).

5. An ultraviolet liquid treatment apparatus as claimed in claim 1 wherein said ballast (8) is provided within said lamp protecting pipe (4) and has attached thereto a socket (8a) for connection with said ultraviolet lamp (5).

6. An ultraviolet liquid treatment apparatus as claimed in claim 1 wherein said ballast (8) is provided at and projects from one end of said lamp protecting pipe (4) to contact the liquid to be treated, and where the one end of said lamp protecting pipe (4) is fixed in place via said ballast (8).
